# EUROPEAN PATENT APPLICATION

(11) **EP 4 265 112 A1**
(43) Date of publication of application: **25.10.2023**
(21) Application number: 23167621.4
(22) Date of filing: 12.04.2023
(51) Int. Cl.: A21C 1/00, A21C 11/00, A23L 7/109, F26B 15/12, F26B 25/22, G01N 22/04

(54) **DRYING DEVICE, PARTICULARLY FOR THE PASTA INDUSTRY**

(30) Priority: 20.04.2022 IT 202200007793
(71) Applicant: R A M Elettronica S.r.l., 76123 Andria (BA) (IT)
(72) Inventor: SCARCELLI, Riccardo, 76123 ANDRIA (BT) (IT)
(74) Representative: Corradini, Corrado

(57) **Abstract**

The present invention relates to a drying device (1), particularly for the pasta industry, comprising a dryer assembly (2) provided with a unit for the control and management (9) of the drying parameters, such as speed of advancement and drying temperature, and with a station for the accumulation (3) of the dried product (4) which is adapted to accommodate temporarily a bundle of dried product (4), of predetermined quantity, in output the dryer assembly (2) and is adapted to unload it onto a movement line (5) that is adapted to transport the dried product (4) away from the drying device (1), for example towards an external packaging machine.

The peculiarity of the invention consists in the fact of comprising moisture sensor means (8) arranged substantially in output from the dryer assembly (2) and functionally connected to said control and management unit (9) in order to vary in real time the drying parameters of the dryer assembly (2) on the basis of the moisture measurement of the dried product (4) performed by the sensor means (8).

## Description

The present invention relates to a drying device, particularly for the pasta industry.

In the pasta industry, the production process involves the use of dryers that are suitable for modulating the amount of water in the pasta, to an optimal value, before it is packaged. In fact, it is evident that an incorrect amount of water in the product can damage it and/or alter its organoleptic characteristics.

Think of, for example, bacterial proliferation or the rotting thereof, in the case of excessively high amounts of water.

It is therefore known to use moisture measurement instruments used for the sample measurement of a batch of product in order to discreetly correct the drying parameters. In more detail, there are direct and indirect measurement techniques of the possessed moisture.

The first ones are destructive techniques with which the measurement of the mass of water present in the sample is carried out, which is taken off the line and no longer reintroduced into the same.

In this case, the drying process does not stop until an anomaly is detected in the sample taken which leads to the rejection of the entire dried batch with the selected parameters. The second ones, on the other hand, are techniques that measure a physical property of the product deriving from or correlated to the moisture contained therein.

In this case, the product can be analysed in-line and quickly.

Typically, such measurement techniques are implemented with transducers operating directly in the drying process.

Although in theory these second techniques allow, as already mentioned, to monitor in real time the moisture value present in the product at a certain point of the drying process, they present a certain degree of error due to the fact that the product is moving inside the dryer and therefore the measurement might not be totally reliable as it depends on the positioning and on the speed of the product itself.

Moreover, considering that, after the measurement has been carried out, the product undergoes the final part of the drying process, it is not possible to have an exact feedback of the variation in moisture inside the product during that final part of the drying process. All this implies an important burden from the point of calibration of the instruments used with complex calculation algorithms.

The main task of the present invention consists in realizing a drying device, particularly suitable for the pasta industry, which overcomes the limits and drawbacks complained about above.

Within the scope of this task, an object of the present invention consists in realizing a drying device that offers the highest guarantees of reliability and efficiency.

Another object of the present invention consists in realizing a drying device that can be realized with technologies known per se and that therefore has low realization costs.

The above task, as well as the purposes mentioned and others that will better appear later, are achieved by a drying device, particularly for the pasta industry, comprising a dryer assembly provided with a unit for the control and management of the drying parameters and with a station for the accumulation of the dried product which is adapted to accommodate temporarily a bundle of said dried product in output from said dryer assembly and is adapted to unload said bundle onto a movement line that is adapted to transport said dried product away from said drying device, for example towards a packaging machine; characterized in that it comprises moisture sensor means (i.e. configure to measure the moisture of the dried products) wherein the sensor means are arranged substantially in output from said dryer assembly and are functionally connected to said control and management unit in order to vary in real time the drying parameters of said dryer assembly on the basis of the moisture measurement of said dried product performed by said sensor means.

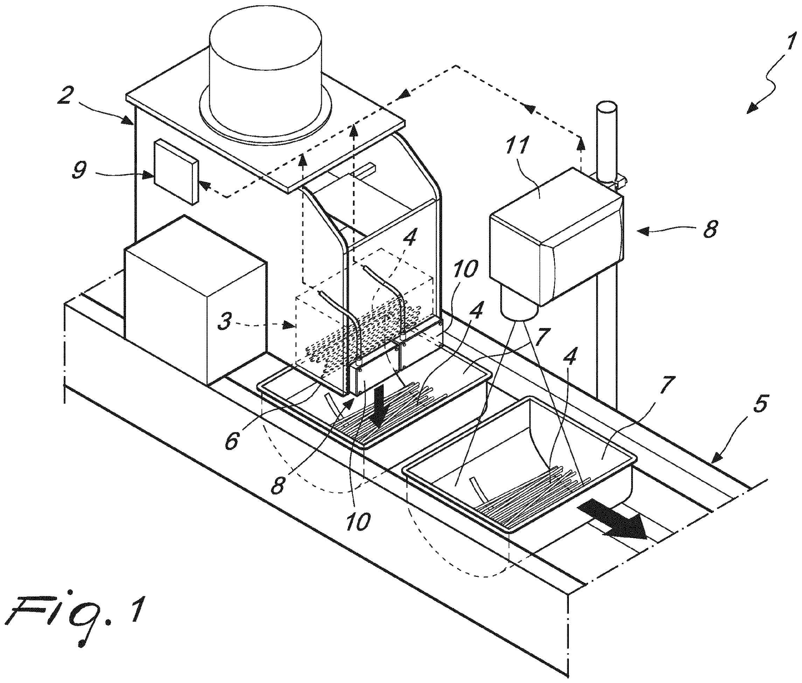

Further features and advantages will become clearer from the description of preferred, but not exclusive, embodiment of a drying device, particularly for the pasta industry, illustrated merely by way of non-limiting example with the aid of the accompanying drawings in which Figure 1 is a schematic perspective view of the drying device according to the present invention.

With reference to the cited figure, the drying device, particularly for the pasta industry, globally indicated with reference numeral 1, comprises a dryer assembly 2 provided with a unit for the control and management 9 of the drying parameters, firstly among them the speed of advancement of the product in the dryer assembly and the drying temperature, and a station for the accumulation 3 of the dried product 4, for example pasta, which is adapted to accommodate temporarily a bundle of dried product 4 in output from the dryer assembly 2 and is adapted to unload said bundle onto a movement line 5 that is adapted to transport the dried product 4 away from the drying device 1, for example towards a packaging machine.

As regards the advancement line 5, in the proposed embodiment, this may be of the traditional type, for example with conveyor belt and/or with catenary, and is positioned below the unloading mouth 6 of the dryer assembly 2 in such a manner as to receive the dried product 4 by gravity after opening a hatch located at the bottom of the accumulation station 3.

In more detail, each bundle of dried product 4 accumulated in the accumulation station 3 is, by gravity, loaded onto a box 7 that is managed by the advancement line 5 away from the drying device 1.

According to the invention, the drying device 1 comprises moisture sensor means 8 arranged substantially in output from the dryer assembly 1 and functionally connected to the control and management unit 9 in order to vary in real time the drying parameters of the dryer assembly 2 on the basis of the moisture measurement of the dried product 4 performed by the sensor means 8.

In more detail, the sensor means 8 comprise at least one electromagnetic wave transducer 10, for example microwave, installed directly on the accumulation station 3 for measuring the moisture of the dried product 4 during the time period between the completion of the filling of the accumulation station 3 and the operations for unloading it. Suitably, the electromagnetic wave transducer 10 is installed on the accumulation station 3 in such a manner as to be substantially in contact with the dried product 4.

More specifically, the electromagnetic wave transducer 10 is of the microwave type and is configured to emit electromagnetic waves with frequencies substantially comprised between 300 MHz and 300 Ghz and preferably equal to 2.4 Ghz.

The frequency of 2.4 Ghz is the frequency at which the (maximum) peak absorption of the electromagnetic waves by water (contained in the dried product 4) can be observed, therefore it is the optimal frequency to maximize the accuracy of the measurement carried out by the electromagnetic wave transducer 10.

If the accumulation station 3 has more than one accommodation compartment for the dried product or the latter has an elongated geometric shape, such as spaghetti, several electromagnetic wave transducers 10 can be provided for each accommodation compartment present in the accumulation station 3 or aligned along the development line of the dry product 4.

In practice, it can be provided that the accumulation station 3 has a (single) accommodation compartment (for accommodating products having an elongated shape), in this case it is possible to provide more than one electromagnetic wave transducers 10 aligned along the development line of the dried product 4, or, it can be provided that the accumulation station 3 has more than one accommodation compartment, in which case more than one electromagnetic wave transducers 10 (for example one or more) can be provided for each accommodation compartment present in the accumulation station 3 (if more than one is provided for each accommodation compartment then they are arranged aligned along the development line of the dried product 4 contained in the respective compartment).

In combination with or in place of the electromagnetic wave transducers 10, the sensor means 8 comprise at least one infrared transducer 11 which can be installed directly on the movement line 5 in order to measure the moisture of the dried product 4 during the transport of the dried product 4 away from the drying device 1.

More specifically, the infrared transducer 11 is installed on the movement line 5 in such a manner as to detect the reflected radiation of the dried product 4 and is located above it at a distance comprised between 20 and 40 cm.

This distance is the optimal distance to be measured (and is, for example, a function of the type of infrared transducer 11 used) to maximize the accuracy of the measurement made by the infrared transducer 11.

In this way, the content of each box 7 moving on the advancement line 5 is analysed by the infrared transducer 11.

The operation of the drying device 1 is described below.

As the dryer assembly 2 dries the product 4, it is accumulated in the accumulation station 3 until a predetermined quantity is reached.

Once the expected amount has been reached, the product bundle stays a few seconds in the accumulation station 3 remaining in contact with the surrounding walls and, therefore, with the electromagnetic wave transducers 10 that directly face inside the accumulation station 3.

In this period of time, the electromagnetic wave transducers 10 take the measurement and send it to the control and management unit 9 that processes the signal and identifying, in real time, any corrections of the drying walls of the dryer assembly 2. Subsequently, the dried product 4 is loaded by gravity on the underlying boxes 7 that are moving on the advancement line 5 arriving at the infrared transducer 11, if provided, which performs the measurement in combination with or in place of the electromagnetic wave transducers 10 and then sends it to the control and management unit 9 which, as already mentioned, processes the signal identifying, in real time, any corrections of the drying walls of the dryer assembly 2.

Appropriately, the signals made by the sensor means 8 are processed by special calculation algorithms that allow to view, through a special monitor, simultaneously all the measurements made and to modify all the main parameters of the sensor means, as well as to define the calibration parameters.

For example, the electronic control and management unit 9 can be configured to receive, as input, the signals made by the sensors (indicative of the moisture measurements contained by the dried products 4) and transmit to the monitor as output (in addition to all the measurements made) also an information (perceptible by a human being) indicative of an anomaly to be corrected, for example by modifying (manually or semi-automatically - with human consent -) some or all of the drying parameters of the dryer assembly 2. In practice, the electronic control and management unit 9 can be configured to suggest a modification/correction of some or all of the drying parameters of the dryer assembly 2 on the basis of the moisture values of the dried products measured by the sensor means 8.

Alternatively or additionally, it is possible to provide that the control and management unit 9 can be configured to perform an automatic feedback control to modify/correct some or all of the drying parameters of the dryer assembly 2 on the basis of the moisture values of the dried products measured by the sensor means 8.

For example, the control and management unit 9 may be configured to:
- measure an (average) moisture value of the dried product;
- modify/correct some or all of the drying parameters of the dryer assembly 2, for example (command the dryer assembly to) decrease the advancement speed of the product in the dryer assembly 2 and/or increase the drying temperature in the dryer assembly 2), if the (average) measured moisture value is greater than a predetermined reference value thereof.

Alternatively, the control and management unit may be configured to:
- measure an (average) moisture value of the dried product;
- modify/correct some or all of the drying parameters of the dryer assembly 2, for example determine a correct value of a drying parameter chosen from the group consisting of the advancement speed of the product in the dryer assembly and the drying temperature in the dryer assembly 2, such as the output of said calculation algorithm which receives as input the measured moisture value of the dried product.

In addition, the moisture measurement, detected by each transducer, measured by the average over the time interval in which the dried product 4 is present near the sensor means 8 can also be displayed on the monitor.

If the electromagnetic wave transducers 10 do not detect the presence of the dried product 4, or the latter should be out of the field of view of the infrared transducer 11, the control and management unit 9, which contains the operating software of the drying device 1, discards the measurements obtained.

When, on the other hand, the dried product 4 is detected, the control and management unit 9 performs a moving average whose final value is displayed as soon as the dried product 4 is unloaded into the boxes 7, in the case of the electromagnetic wave transducers 10, or leaves the field of view of the infrared transducers 11.

The discriminant between the presence of the dried product 4 and the absence thereof is obtained on the basis of the interpretation of the data measured by means of a threshold value.

This interface therefore shows a value each time that a suitable amount of dried product 4 passes in the vicinity of the sensor means 8, typically at each unloading or at each full box 7.

In practice, it has been found that the drying device, particularly for the pasta industry, according to the present invention, fulfils the task as well as the intended purposes.

In fact, thanks to the fact that the sensor means are located in output from the dryer assembly, they can perform a reliable measurement simplifying the calibration phases. The measurement performed therefore allows to handle the drying parameters in real time.

This results in an optimization of the entire process, reduction of waste and standardization of the product in terms of organoleptic properties and of preservation. The drying device, particularly for the pasta industry, thus conceived, is susceptible to numerous modifications and variants all falling within the scope of the inventive concept. Furthermore, all the details can be replaced by other technically equivalent elements.

In practice, any materials can be used according to requirements, as long as they are compatible with the specific use, the dimensions and the contingent shapes.

## Claims

1. A drying device (1), particularly for the pasta industry, comprising comprising a dryer assembly (2) provided with a unit for the control and management of the drying parameters and with a station (3) for the accumulation of the dried product (4) which is adapted to accommodate temporarily a bundle of said dried product (4) in output from said dryer assembly (2) and is adapted to unload said bundle onto a movement line (5) that is adapted to transport said dried product (4) away from said drying device (1); **characterized in that** it comprises moisture sensor means (8) configured to measure the moisture of the dried product (4), wherein the sensor means (8) are arranged in output from said dryer assembly (2) and are functionally connected to said control and management unit, in order to vary in real time the drying parameters of said dryer assembly (2) on the basis of the moisture measurement of said dried product (4) performed by said sensor means (8).

2. The drying device (1) according to claim 1, **characterized in that** said sensor means (8) comprise at least one electromagnetic wave transducer (10) installed directly in said accumulation station (3) in order to measure the moisture of said dried product (4) during the time period between the completion of the filling of said accumulation station (3) and the operations for unloading said accumulation station (3).

3. The drying device (1) according to claim 2, wherein said at least one electromagnetic wave transducer (10) is installed on said accumulation station (3) so as to be in contact with said dried product (4).

4. The drying device (1) according to claim 2, **characterized in that** said at least one electromagnetic wave transducer (10) is configured to emit electromagnetic waves with frequencies substantially comprised between 300 MHz and 300 GHz.

5. The drying device (1) according to claim 4, **characterized in that** said at least one electromagnetic wave transducer (10) is configured to emit electromagnetic waves with a frequency preferably equal to 2.4 GHz.

6. The drying device (1) according to one or more of the preceding claims, **characterized in that** said accumulation station (3) comprises more than one accommodation compartment (3) for the dried product and, wherein said at least one electromagnetic wave transducer (10) comprises one electromagnetic wave transducer (10) for each accommodation compartment that is present in said accumulation station (3).

7. The drying device (1) according to one or more of the preceding claims, **characterized in that** said sensor means (8) comprise at least one infrared ray transducer (11) which can be installed directly on said movement line (5) in order to measure the moisture of said dried product (4) during the transport of said dried product (4) away from said drying device (1); said at least one infrared ray transducer (11) being installed on said movement line (5) in such a manner as to detect the reflected radiation of said dried product (4).

8. The drying device (1) according to claim 7, **characterized in that** said at least one infrared ray transducer (11) is located above said movement line (5) at a distance comprised between 20 and 40 cm.
